(19) Europäisches Patentamt
European Patent Office
Office européen des brevets

(11) EP 3 663 386 A1

(12) # EUROPÄISCHE PATENTANMELDUNG

(43) Veröffentlichungstag:
**10.06.2020 Patentblatt 2020/24**

(21) Anmeldenummer: **18210474.5**

(22) Anmeldetag: **05.12.2018**

(51) Int Cl.:
***C11D 1/66*** *(2006.01)* ***A61K 8/37*** *(2006.01)*
***A61K 8/99*** *(2017.01)* ***C11D 1/825*** *(2006.01)*
***C11D 1/83*** *(2006.01)* ***C11D 1/835*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **LANXESS Deutschland GmbH**
**50569 Köln (DE)**

(72) Erfinder:
- **Henkel, Dr. Thomas**
  **44135 Dortmund (DE)**
- **Bitzer, Dr. Jens**
  **44225 Dortmund (DE)**

(54) **GLYKOLIPID ENTHALTENDE TENSIDMITTEL**

(57) Die Erfindung betrifft Mittel enthaltend spezielle Glykolipid-Biotenside und weitere Tenside, die Verwendung dieser Mittel zur Reinigung von Oberflächen und ein Verfahren zur Reinigung von Oberflächen mit den erfindungsgemäßen Mitteln.

EP 3 663 386 A1

## Beschreibung

Die Erfindung betrifft Mittel enthaltend spezielle Glykolipid-Biotenside und weitere Tenside, die Verwendung dieser Mittel zur Reinigung von Oberflächen und ein Verfahren zur Reinigung von Oberflächen mit den erfindungsgemäßen Mitteln.

Die speziellen Glykolipid-Biotenside haben neben tensidischen auch vorteilhafte antimikrobielle Eigenschaften, die u.a. aus der WO-A 2012/167920 bekannt sind.

Aus dem Stand der Technik sind Reinigungsmittel bekannt, die verschiedene bekannte Glykolipide als Biotenside enthalten, die sich von den hier beschriebenen speziellen Glykolipid-Biotensiden in ihrer chemischen Struktur unterscheiden.

Glykolipid-Biotenside, wie auch die hier beschriebenen speziellen Glykolipid-Biotenside, werden biotechnologisch aus Mikroorganismen gewonnen und gelten als umweltschonende, nachhaltig produzierte und sichere Alternativen zu chemisch hergestellten Tensiden aus fossilen Grundstoffen.

Aus der EP-A 3290500 sind Wasch-, Pflege- und Reinigungsmittel bekannt, die alkoxylierte Tenside und mindestens ein Glykolipid-Biotensid enthalten.

Aus der WO-A 2015091250 sind wässrige Reinigungsmittel bekannt, die Glykolipid-Biotenside und ein weiteres Tensid enthalten und bei Kontakt mit Ölen und/oder Fetten spontan Mikroemulsionen bilden.

Reinigungszusammensetzungen, die ein synthetisches Tensid und ein Glykolipid-Biotensid enthalten, sind aus der EP-B 2988829 bekannt.

Die EP-B 2931237 beschreibt Körperpflegezusammensetzungen, enthaltend synthetische anionische Tenside, Glykolipid-Biotenside und ein Fettacylisothionat-Produkt in bestimmten Mengenverhältnissen.

Waschmittelzusammensetzungen, enthaltend ein Glykolipid-Biotensid mit micellarer Phase und ein zweites Glykolipid-Biotensid ausgewählt aus der Gruppe Rhamnolipiden, Glucoselipiden, Trehaloselipiden und Gemischen dieser Glykolipide oder einem Nichtglykolipidtensid sind aus der EP-B 0499434 bekannt.

Nachteilig an allen diesen Mitteln und Mischungen ist weiterhin, dass die Reinigungsleistung nicht zufriedenstellend ist.

Es bestand daher weiterhin ein Bedürfnis nach einem Reinigungsmittel, mit dem die Nachteile des Standes der Technik überwunden werden können und eine hohe Reinigungsleistung erzielt wird.

Überraschend wurde jetzt gefunden, dass die speziellen Glykolipid-Biotenside dieser Anmeldung in Kombination mit einem weiteren Tensid Mittel darstellen, die eine hohe Reinigungsleistung aufweisen.

Gegenstand der Erfindung sind daher Mittel enthaltend,

(i) mindestens eine Verbindung der Formel (I)

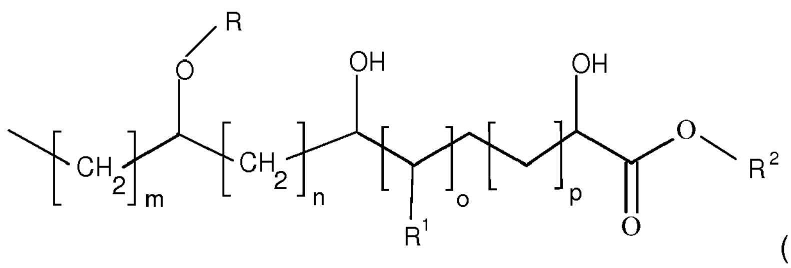

(I)

mit

m = 3 bis 5, bevorzugt 3 oder 5;

n = 2 bis 5, bevorzugt 3;

o = 0 oder 1 und

$R^1$ = H oder OH

$R^2$ = ist -H, Alkalimetall- oder Erdalkalimetallkation oder -$C_1$-$C_6$-Alkyl; vorzugsweise -H oder Alkalimetall- oder Erdalkalimetallkation und

p = 3 bis 17, bevorzugt 5 bis 15 wobei die Summe von m + n + o + p nicht kleiner als 14 ist und

R einen Kohlenhydratrest darstellt, der über ein Kohlenstoffatom an dem Sauerstoffatom gebunden ist

oder Gemische der Verbindungen der Formel (I) und

(ii) mindestens ein weiteres Tensid.

Bevorzugt stellt der Kohlenhydratrest R ein Trisaccharid dar. Besonders bevorzugt enthält dieses Trisaccharid mindestens eine Xylopyranoseeinheit und/oder mindestens einen Glucopyranoseeinheit.

Bevorzugt stellt der Kohlenhydratrest R einen Rest der Formel (II) dar

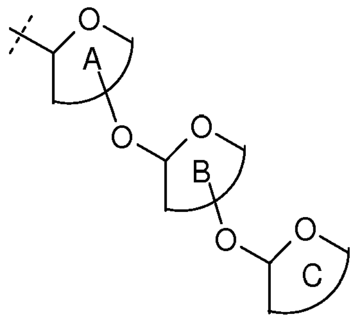

(II)

bei dem die Ringe, A, B und C unabhängig voneinander Monosaccharidreste sind, mit mindestens einer Hydroxylgruppe, die gegebenenfalls acyliert sein können. Bevorzugt sind die Ringe A und B Xylopyranosereste und der Ring C ist ein Glucopyranoserest.

Die Verbindungen der Formel (I) können in Form eines Esters vorliegen und eine oder mehrere funktionelle -(C=O)-O- Gruppen enthalten. Vorzugsweise enthalten die Verbindungen der Formel (I) mehr als eine Estergruppe. Ganz besonders bevorzugt enthalten die Verbindungen der Formel (I) zwei Estergruppen.

Die Ester können auch intramolekular, in Form eines Lactons, vorliegen. In diesem Fall bildet sich zwischen der Carboxylfunktion des linearen Fettsäurerestes und einer terminale Hydroxylgruppe des Kohlenhydratrestes R eine Esterbindung aus.

Alternativ oder zusätzlich kann jede der Hydroxylgruppen des Kohlenhydratrestes R oder/und des linearen Fettsäurerestes durch eine Carbonsäure verestert sein. Vorzugsweise handelt es sich um eine aliphatische Carbonsäure. Besonders bevorzugt handelt es sich um eine aliphatische Carbonsäure mit einem $C_3$-$C_{10}$-Alkylrest. Ganz besonders bevorzugt handelt es sich bei der Carbonsäure um Isovaleriansäure.

In einer weiteren Ausführungsform der Erfindung sind 1, 2 oder 3 Hydroxylgruppen des Kohlenhydratrestes R der Formel (I) oder/und des linearen Fettsäurerestes durch eine Carbonsäure verestert. Vorzugsweise handelt es sich um eine aliphatische Carbonsäure. Besonders bevorzugt handelt es sich um eine aliphatische Carbonsäure mit einem $C_3$-$C_{10}$-Alkylrest. Ganz besonders bevorzugt handelt es sich bei der Carbonsäure um Isovaleriansäure.

In einer weiteren Ausführungsform der Erfindung ist mindestens eine der Hydroxylgruppen des Kohlenhydratrestes R der Formel (I) mit einer Carbonsäure mit mindestens drei Kohlenstoffatomen verestert. Bevorzugt ist mindestens eine der Hydroxylgruppen des Kohlenhydratrestes R der Formel (I) mit einer Isovalerylgruppe verestert.

Besonders bevorzugt ist der Kohlenhydratrest R eine Verbindung der Formel (III)

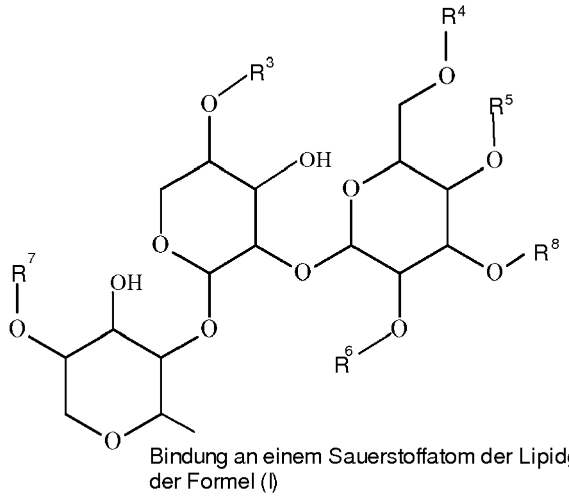

Bindung an einem Sauerstoffatom der Lipidgruppe der Formel (I)

(III)

wobei $R^3$, $R^4$, $R^5$, $R^6$ und $R^7$ unabhängig voneinander -H oder $-C(=O)C_1-C_{12}$-Alkyl sind und $R^8$= H, $-C(=O)-CH_2-C((CH_3)(OH))-CH_2-COOH$ oder $-C(=O)C_1-C_{12}$-Alkyl ist; wobei $R^3$, $R^4$, $R^5$, $R^6$ $R^7$ und $R^8$ bevorzugt unabhängig voneinander -H oder $-C(=O)C_1-C_6$-Alkyl sind ist, wobei $R^3$, $R^4$, $R^5$, $R^6$ und $R^7$ besonders bevorzugt unabhängig voneinander -H oder - $C(=O)C_3-C_6$-Alkyl und $R^8$ bevorzugt H oder $-C(=O)C_1-C_6$-Alkyl sind, noch weiter bevorzugt sind $R^4$ = -C(=O)isobutyl und $R^3$, $R^5$ , $R^6$, $R^7$ und $R^8$ unabhängig voneinander =-H oder/und $-C(=O)CH_3$.

In einer weiteren Ausführungsform der Erfindung ist mindestens eine Verbindung der Formel (I) eine Verbindung der Formel (IV)

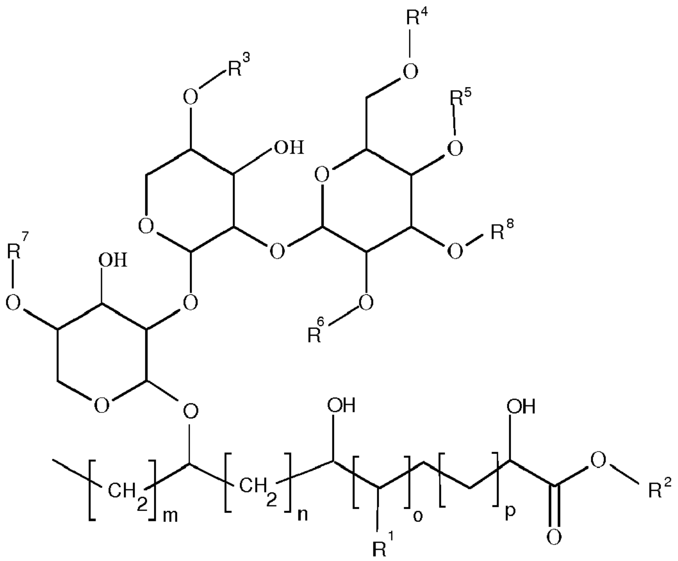

(IV)

wobei,

m = 4 oder 6,

n = 3,

o = 1

p = 11 oder 13

$R^1$ ist -H oder -OH, bevorzugt ist $R^1$ = OH,

$R^2$ ist -H, Alkalimetall- oder Erdalkalimetallkation oder -$C_1$-$C_6$-Alkyl; vorzugsweise -H oder Alkalimetall- oder Erdalkalimetallkation; und

$R^3$, $R^4$, $R^5$, $R^6$ und $R^7$ sind unabhängig voneinander -H oder -C(=O)$C_1$-$C_{12}$-Alkyl

und $R^8$= H, -C(=O)-$CH_2$-C(($CH_3$)(OH))-$CH_2$-COOH oder -C(=O)$C_1$-$C_{12}$-Alkyl ist.

In einer bevorzugten Ausführungsform der Erfindung sind $R^3$, $R^4$, $R^5$, $R^6$ ,$R^7$ und $R^8$ unabhängig voneinander -H oder -C(=O)$C_1$-$C_6$-Alkyl, weiter bevorzugt -H oder -C(=O)$C_3$-$C_6$-Alkyl und $R^8$ = -H oder -C(=O)$C_1$-$C_6$-Alkyl, noch weiter bevorzugt sind $R^4$ = - C(=O)isobutyl und $R^3$, $R^5$, $R^6$, $R^7$, $R^8$ = -H oder/und -C(=O)$CH_3$.

In einer weiteren bevorzugten Ausführungsform sind $R^2$, $R^6$ und $R^7$ = -H.

In einer weiteren bevorzugten Ausführungsform ist die Verbindung der Formel (I) mindestens eine Verbindung der Formel (V-A) bis (V-D) oder Gemische dieser Verbindungen

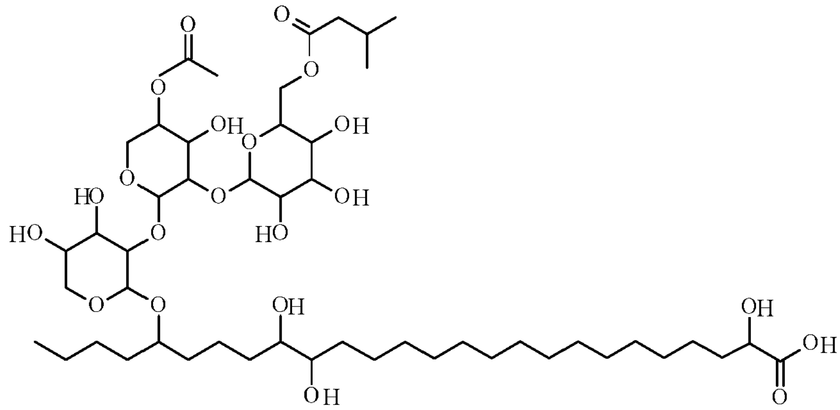

(V-A)

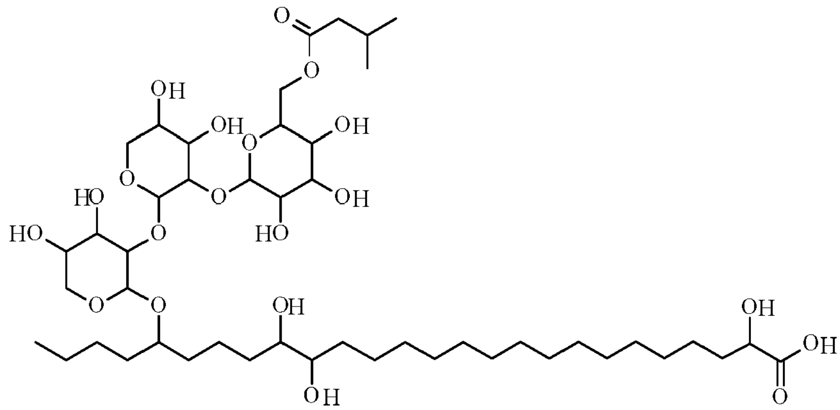

(V-B)

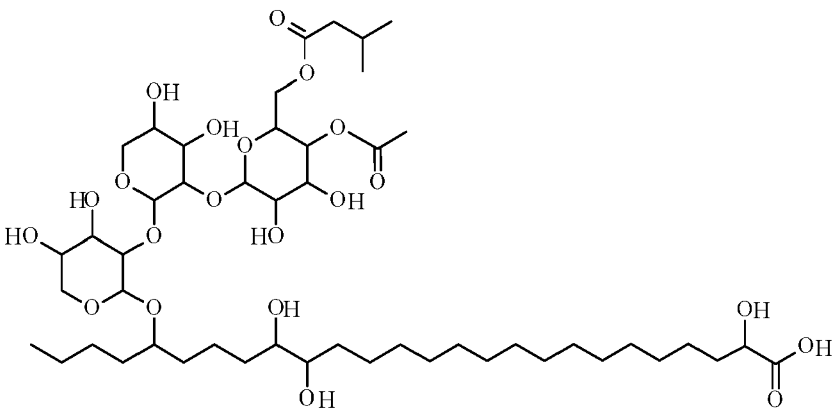

(V-C)

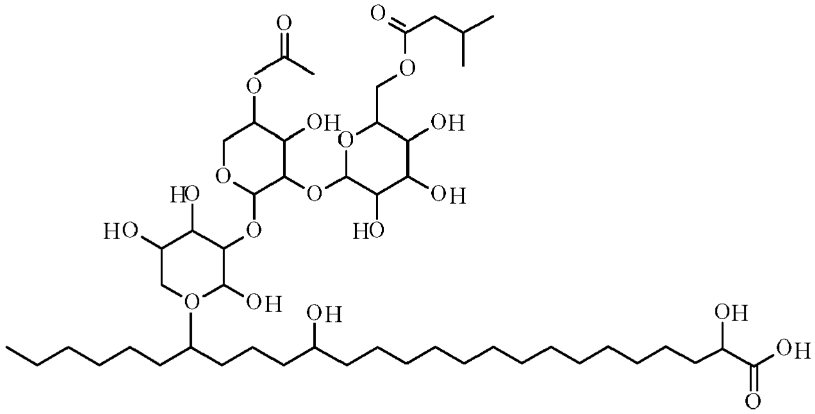

(V-D)

und Salze dieser Verbindungen.

In einer weiteren bevorzugten Ausführungsform sind Salze im Sinne der Erfindung Alkalimetal- und/oder Erdalkalimetalsalze, besonders bevorzugt Alkalimetallsalze.

Die Verbindungen der Formel (V) sind spezielle Glykolipid-Biotenside.

Die Verbindungen der Formeln (I) bis (V) werden durch Fermentation aus Pilzen gewonnen. Die Herstellung dieser Verbindungen ist z.B. aus der WO-A 2012/167920 bekannt. Vorzugsweise werden zur Herstellung der Verbindungen der Formeln (I) bis (V) Pilze der Familien *Dacrymycetaceae*, bevorzugt der Gattungen *Dacryopinax*, *Ditiola*, *Guepiniopsis* and/or *Femsjonia*, besonders bevorzugt der Art *Dacryopinax spathularia*, *Dacrymyces sp., Dacrymyces stillatus, Dacrymyces chrysocomus, Guepiniopsis buccina* und/oder *Femsjonia luteo-alba* (= *Ditiola pezizaeformis*) eingesetzt. Besonders bevorzugt werden die Verbindungen der Formeln (I) bis (V) aus Pilzen der Art *Dacryopinax spathularia* des Stammes MUCL 53181, *Dacryopinax spathularia* des Stammes MUCL 53182, *Ditiola radicata* des Stammes MUCL 53180, *Ditiola nuda* des Stammes MUCL 53179, *Dacrymyces chrysocomus* des Stammes CBS280.84 and *Femsjonia luteo-alba* (= *Ditiola pezizaeformis*) des Stammes MUCL 53500 hergestellt. Die Verbindungen der Formel (I) werden vorzugsweise aus den Pilzen als Gemische in Form eines Extraktes gewonnen. Diese können dann weiter aufgereinigt werden, z.B. durch Säulenchromatographie, wie insbesondere durch Hochdrucksäulenchromatographie oder durch Filtration und Fällung. Die Aufreinigungsverfahren sind dem Fachmann, z.B. aus der WO-A 2012/167920, bekannt.

In einer weiteren Ausführungsform der Erfindung wird zur Herstellung der Verbindungen der Formel (I) bis (V) der Pilz *Dacryopinax sphatularia* des Stammes MUCL 53181 verwendet.

In einer weiteren Ausführungsform der Erfindung werden die Verbindungen der Formel (I) als Gemische eingesetzt.

In einer weiteren Ausführungsform der Erfindung werden Gemische der Verbindungen der Formel (V-A), Formel (V-B), Formel (V-C) und der Verbindungen der Formel (V-D) eingesetzt. Bevorzugt werden Gemische der Verbindungen der Formel (V-A) und Verbindungen der Formel (V-B) und Verbindungen der Formel (V-C) und Verbindungen der Formel (V-D) in einer Menge von 30 Gew. % bis 95 Gew. % bezogen auf die Gesamttrockenmasse der eingesetzten Verbindungen der Formel (I) eingesetzt. Besonders bevorzugt werden Gemische der Verbindungen der Formel (V-A) und Verbindungen der Formel (V-B) und Verbindungen der Forme (V-C) und Verbindungen der Formel (V-D) in einer Menge von 40 Gew. % bis 60 Gew. % bezogen auf die Gesamttrockenmasse der eingesetzten Verbindungen der Formel (I) eingesetzt. Die restlichen Verbindungen in den Gemischen können Salze oder weitere Verbindungen der Formel (I) sein.

Tenside ii) im Rahmen dieser Erfindung sind vorzugsweise Verbindungen, die die Fähigkeit besitzen, die Oberflächenspannung von Wasser bei 20°C und bei einer Konzentration von 0.5 Gew.-%, bezogen auf die Gesamtmenge der tensidischen Zubereitung, auf unter 45 mN/m zu verringern.

Als Tenside ii) können Glykolipide, anionische Tenside, nicht-ionische Tenside, kationische Tenside oder amphotere Tenside eingesetzt werden.

Als Glykolipide werden bevorzugt Rhamnolipide, insbesondere Mono-, Di- oder Polyrhamnolipide, Sophorolipide in ihrer Säure- oder Laktonform oder als deren Gemische, diacetyliert, acetyliert oder nicht-acetyliert, Trehaloselipide, Mannosylerythritollipide, Glucoselipide (Alkylglucoside) und Cellulobioselipide eingesetzt.

Als anionische Tenside eignen sich vorzugsweise $C_8$-$C_{18}$-Alkylbenzolsulfonate, wie insbesondere Natriumdodecylsulfat (CAS: 25155-30-0), $C_8$-$C_{20}$ Alkylsulfonate, $C_8$-$C_{18}$-Monoalkylsulfate, $C_8$-$C_{18}$-Alkylpolyglykolethersulfate mit 2 bis 6 Ethylenoxideinheiten (EO) im Etherteil, sowie Sulfobernsteinsäuremono- und -di-$C_8$-$C_{18}$-Alkylester. Weiterhin werden vorzugsweise $C_8$-$C_{18}$- -Olefinsulfonate, sulfonierte $C_8$-$C_{18}$-Fettsäuren, insbesondere Dodecylbenzolsulfonat, $C_8$-$C_{22}$-Carbonsäureamidethersulfate, $C_8$-$C_{18}$-Alkylpolyglykolethercarboxylate, $C_8$-$C_{18}$-N-Acyltauride, N-Acylaminosaurederivate, wie vorzugsweise N-Acylaspartat oder N-Acylglutamat, Methylestersulfonate, N-Sorbitansäureester, $C_8$-$C_{18}$-N-Sarkosinate und $C_8$-$C_{18}$-Alkylisethionate und deren Mischungen verwendet. Besonders bevorzugt werden Natriumdodecylsulfat (CAS: 25155-30-0) und Natriumbutylestersulfat (CAS: 15826-161) oder Mischungen dieser beiden als anionische Tenside eingesetzt.

Die anionischen Tenside werden vorzugsweise als Natriumsalze eingesetzt, können aber auch als andere Alkali- oder Erdalkalimetallsalze, beispielsweise Magnesiumsalze, sowie in Form von Ammonium- oder Mono-, Di-, Tribzw. Tetraalkylammoniumsalzen eingesetzt werden. Im Falle der Sulfonate werden diese vorzugsweise in Form ihrer korrespondierenden Säure, z.B. als Dodecylbenzolsulfonsäure, eingesetzt.

Als nicht-ionische Tenside werden vorzugsweise Fettalkoholpolyglycolether (Fettalkoholalkoxylate), wie besonders bevorzugt $C_8$-$C_{18}$-Alkoholpolyglykolether, wie ganz besonders bevorzugt ethoxylierte und/oder propoxylierte Alkohole mit 8 bis 18 C-Atomen im Alkylteil und 2 bis 15 Ethylenoxid- (EO) und/oder Propylenoxideinheiten (PO), $C_8$-$C_{18}$-Carbonsäurepolyglykolester mit 2 bis 15 EO, vorzugsweise Talgfettsäureester mit 6 Mol Ethylenoxid, ethoxylierte $C_{12}$-$C_{18}$-Fettsäureamide mit 2 bis 8 EO, Aminoxide, $C_8$-$C_{14}$-Alkylpolyglycoside mit 1 bis 3 Glycosideinheiten, wie besonders bevorzugt Decylglucoside (CAS 68515-73-1), wie sie z.B. unter dem Namen TRITON™ der Firma Dow Chemicals bekannt sind, eingesetzt. Ganz besonders bevorzugt werden als nicht-ionische Tenside Oleyl-Cetyl-Alkoholethoxylate mit 5 EO, Nonylphenolethoxylate mit 10 EO, Laurinsäurediethanolamid, Kokosalkyldimethylaminoxid und Kokosalkylpolyglucosid mit im Mittel 1,4 Glucoseeinheiten eingesetzt. Noch weiter bevorzugt werden als nicht-ionische Tenside Fettalkoholpolyglykolether mit insbesondere 2 bis 8 EO, wie vorzugsweise $C_{10}$- oder $C_{12}$-$C_{14}$-Fettalkohol+4-EO-ether, $C_{14}$-$C_{20}$-Aminoxide, sowie $C_8$-$C_{10}$ Alkylglucoside und Alkylpolyglucoside mit 1 bis 2 Glycosideinheiten und Mischungen dieser Verbindungen eingesetzt. Noch weiter bevorzugt werden als nicht-ionisches Tensid Alkylpolyglucoside, insbesondere Decylglucoside (CAS 68515-73-1) eingesetzt.

Amphotere Tenside sind vorzugsweise Betaine der Formel $(R^i)(R^{ii})(R^{iii})N^+CH_2COO^-$ (VI) in der $R^i$ einen gegebenenfalls durch Heteroatome oder Heteroatomgruppen unterbrochenen $C_8$-$C_{25}$-Alkylrest, vorzugsweise mit 10 bis 21 Kohlenstoffatomen, und $R^{ii}$ sowie $R^{iii}$ gleichartige oder verschiedene $C_1$-$C_3$-Alkylreste sind. Besonders bevorzugt sind amphotere Tenside $C_{10}$-$C_{18}$-Alkyldimethylcarboxymethylbetain und $C_{11}$-$C_{17}$-Alkylamidopropyldimethylcarboxymethylbetain und Mischungen dieser Verbindungen. Kationische Tenside sind vorzugsweise quaternäre Ammoniumverbindungen der Formel $(R^{iv})(R^v)(R^{vi})(R^{vii})N^+X^-$ (VII), in der $R^{iv}$ bis $R^{vii}$ gleich- oder verschiedenartig sein kann, und unabhängig voneinander $C_8$-$C_{24}$-Alkyl und $C_1$-$C_6$-Alkyl sind und $X^-$ für ein einwertiges Anion, insbesondere ein Halogenid, darstellen. Vorzugsweise stellen mindestens zwei der Reste $R^{iv}$ bis $R^{vii}$ ein $C_8$-$C_{24}$-Alkyl dar und zwei der Reste $R^{iv}$ bis $R^{vii}$ ein $C_1$-$C_6$-Alkyl. Besonders bevorzugt sind die kationischen Tenside Didecyldimethylammoniumchlorid, Alkylbenzyldidecylammoniumchlorid und deren Mischungen.

Tenside ii) sind vorzugsweise in einer Menge von 1 bis 50 Gew. %, besonders bevorzugt in einer Menge von 5 Gew. % bis 40 Gew. % bezogen auf das Gesamtgewicht an Mittel, in dem Mittel enthalten.

Die Verbindungen der Formeln (I) sind vorzugsweise in einer Menge von 1 bis 50 Gew. %, besonders bevorzugt in einer Menge von 1,5 Gew. % bis 25 Gew. % bezogen auf das Gesamtgewicht an Mittel, in dem Mittel enthalten.

Die Mittel können weitere Zusätze, wie Lösungsmittel, Copolymere, Säuren, Basen, hydrophilierende Agenzien, Desinfektionsmittel, pH-Stellmittel, Duft- und Farbstoffe, Puffer, Viskositätsregulatoren, Korrosionsinhibitoren, Komplexbildner, Filmbildner, antimikrobielle Wirkstoffe, Builder, Bleichmittel, Enzyme, optische Aufheller, Antioxidantien, Opacifier, Hydrotrope, Abrasiva, Konservierungsmittel, Oxidationsmittel oder Insektizide sowie Gemische derselben enthalten.

Die Gewichtsmenge dieser Inhaltstoffe sollte vorzugsweise kleiner als 30 Gew.-% bezogen auf die Gesamtgewichtsmenge des eingesetzten Mittels sein. Die Gewichtsmenge dieser Inhaltstoffe ist bevorzugt 0,01 Gew.-% bis 30 Gew.-%, ganz besonders bevorzugt 0,2 Gew.-% bis 15 Gew.-% bezogen auf die Gesamtgewichtsmenge des Mittels.

Als Lösungsmittel werden vorzugsweise Wasser oder gesättigte, verzweigte oder unverzweigte $C_{1}$-$_{20}$-Kohlenwasserstoffe, bevorzugt $C_{2}$-$_{15}$-Kohlenwasserstoffe, mit mindestens einer Hydroxygruppe und gegebenenfalls einer oder mehreren Etherfunktionen (C-O-C), d.h. die Kohlenstoffatomkette unterbrechenden Sauerstoffatomen, eingesetzt. Besonders bevorzugte Lösungsmittel sind - $C_{2}$-$_{6}$-Alkylenglykole deren OH-Funktionalität einseitig mit einem $C_1$-$C_6$-Alkyl verethert sein kann und Poly-$C_2$-$C_3$-alkylenglykolether mit durchschnittlich 1 bis 9 gleichen oder verschiedenen, vorzugsweise gleichen, Alkylenglykolgruppen pro Molekül, sowie einwertige $C_1$-$C_6$-Alkohole. Als Lösungsmittel werden ganz besonders bevorzugt Ethanol, Poly(oxy-1,2-ethanediyl, Butoxydiglycol ($C_4H_9OCH_2CH_2OCH_2CH_2OH$), Butoxyisopropanol, Butoxypropanol, n-Butylalkohol, t-Butylalkohol, Butyloctanol, Diethylenglycol, Dimethoxydiglycol (Bis(2-methoxyethyl)ether), Dimethylether, Dipropylenglycol, wie vorzugsweise 2,2'-Oxydi-1-propanol, 1,1'-Oxydi-2-propanol oder 2-(2-Hydroxypropoxy)-1-propanol, Ethoxydiglycol, Ethoxyethanol, Glycol, Hexylenglycol, Isobutoxypropanol, Isopentyldiol, Isopropylalkohol, 3-Methoxybutanol, Methoxydiglycol, Methoxyethanol, Methoxyisopropanol, Methoxymethylbutanol, Methylal, Methylalkohol, Neopentylglycol, Polyethylenglykol-4 (PEG-4) (CAS 25322-68-3), PEG-6, PEG-7, PEG-8, PEG-9, PEG-6 Methyl Ether, Pentylenglycol, PPG-7, PPG-2-Buteth-3, PPG-2 Butylether, Propandiol, Propylalkohol (n-Propanol), Propylenglycol, Propylenglycolbutylether, Propylenglycolpropylether, Trimethylhexanol, Ethylenglykolmonobutylether, Propylenglykolmonobutylether, Diethylenglykolmonobutylether, Propylenglykolmonotertiärbutylether und Propylenglykolmonoethylether eingesetzt.

Lösungsmittel sind vorzugsweise in dem Mittel in einer Gewichtsmenge von 0,01 bis 30 Gew.-%, vorzugsweise 10 bis 15 Gew.-% bezogen auf die Gesamtgewichtsmenge des Mittels enthalten.

Als Copolymere werden vorzugsweise Polyether-Polyester-Copolymere, auf der Basis alkoxylierter Terephthalsäureester eingesetzt.

Das Polyether-Polyester-Copolymer ist vorzugsweise in einer Gewichtsmenge von 0,1 bis 5 Gew.-% bezogen auf die Gesamtgewichtsmenge des Mittels enthalten.

Als Basen werden vorzugsweise Ammoniak, $C_1$-$C_9$-Alkanolamine, wie besonders bevorzugt Ethanolamin, und Alkali- und Erdalkalimetallhydroxide und -carbonate eingesezt. Als Alkalimetallhydroxide werden vorzugsweise Kaliumhydroxid und Natriumhydroxid eingesetzt.

Der Gehalt an Basen im Mittel beträgt vorzugsweise 0,01 bis 2 Gew.-% bezogen auf die Gesamtgewichtsmenge des Mittels.

Die Mittel können auch von den Verbindungen der Formel (I) verschiedene Säuren, wie vorzugsweise Carbonsäuren, enthalten. Geeignet sind Carbonsäuren mit bis zu 6 C-Atomen, wobei es sich um Mono-, Di- oder Polycarbonsäuren handeln kann. Carbonsäuren sind vorzugsweise Essigsäure, Glykolsäure (alpha-Hydroxycarbonsäure), Milchsäure, Citronensäure, Bernsteinsäure, Adipinsäure, Äpfelsäure, Weinsäure und Gluconsäure. Besonders bevorzugt wird Citronensäure eingesetzt.

Der Gehalt an Säuren ist vorzugsweise 0,01 Gew.-% bis 2,7 Gew.-%, besonders bevorzugt 0,01 Gew.-% bis 0,9 Gew.-% bezogen auf die Gesamtgewichtsmenge des Mittels.

Das erfindungsgemäße Mittel kann auch Mittel zur Hydrophilierung von Oberflächen enthalten. Zur Hydrophilierung werden vorzugsweise kolloidale Silica-Sole, in denen das Siliciumdioxid nanopartikulär vorliegt, eingesetzt. Kolloidale nanopartikuläre Silica-Sole sind stabile Dispersionen von amorphem, partikulärem Siliciumdioxid $SiO_2$ mit Partikelgrößen im Bereich von 1 bis 100 nm.

In einer weiteren Ausführungsform der Erfindung werden zur Hydrophilierung von Oberflächen vorzugsweise amphotere Polymere, wie vorzugsweise Acrylpolymere, wie besonders bevorzugt Copolymere aus Acryl- oder Methacrylsäure und einer polymerisierbaren quaternären Ammoniumverbindung, Copolymere mit 2-Acrylamido-2-methylpropansulfonsäure, Polyethersiloxane, also Copolymere von Polymethylsiloxanen mit Ethylenoxid- oder Propylenoxidsegmenten, Maleinsäure-Copolymere und Polyurethane mit Polyethylenglykol-Einheiten, eingesetzt.

Als Viskositätsregulatoren werden vorzugsweise organische natürliche Verdickungsmittel, wie vorzugsweise Agar-Agar, Carrageen, Tragant, Gummi arabicum, Alginate, Pektine, Polyosen, Guar-Mehl, Johannisbrotbaumkernmehl, Stärke, Dextrine, Cyclodextrine, Gelatine, Casein, organische abgewandelte Naturstoffe, wie vorzugsweise Carboxymethylcellulose und andere Celluloseether, Hydroxyethyl- und -propylcellulose, Kernmehlether, organische Vinylpolymere, Polycarbonsäuren, Polyether, Polyimine, Polyamide, Polykieselsäuren,
Polyacryl- und Polymethacrylpolymere, Polysaccharide und Heteropolysaccharide, Stärke, Schichtsilikate, wie vorzugsweise Magnesium- oder Natrium-Magnesium-Schichtsilikate und Zeolithe eingesetzt. Ein besonders bevorzugter Polysaccharidverdicker ist Xanthan Gum.

In einer bevorzugten Ausführungsform enthält das erfindungsgemäße Mittel, 0,01 Gew.-% bis 30 Gew.-%, insbesondere 0,2 Gew.-% bis 15 Gew.-% eines Verdickers bezogen auf die Gesamtgewichtsmenge des Mittels.

Korrosionsinhibitoren sind vorzugsweise Cyclohexylamin, Diammoniumphosphat, Dilithiumoxalate, Dimethylaminomethylpropanol, Dikaliumoxalat, Dikaliumphosphat, Dinatriumphosphat, Dinatriumpyrophosphat, Nitromethan, Kaliumsilikat, Natriumaluminat, Natriumhexametaphosphat, Natriummetasilikat, Natriummolybdate, Natriumnitrit, Natriumoxalat und Triisopropanolamine.

Komplexbildner auch Sequestriermittel genannt, sind Inhaltsstoffe, die Metallionen zu komplexieren und inaktivieren vermögen, um ihre nachteiligen Wirkungen auf die Stabilität oder das Aussehen der Mittel, beispielsweise Trübungen, zu verhindern.

Bevorzugt sind die folgenden gemäß *INCI (International Nomenclature of Cosmetic Ingredients)* bezeichneten Komplexbildner: Aminotrimethylene Phosphonic Acid, Beta-Alanine Diacetic Acid, Calcium Disodium EDTA, Citric Acid, Cyclodextrin, Cyclohexanediamine, Tetraacetic Acid, Diammonium Citrate, Diammonium EDTA, Diethylenetriamine Pentamethylene Phosphonic Acid, Dipotassium, EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene, Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphosphate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Bevorzugt werden als Filmbildner filmbildenden Polymere eingesetzt vorzugsweise ausgewählt aus der Gruppe umfassend Polyethylenglykol, Polyethylenglykol-Derivate sowie Gemische derselben, vorzugsweise mit einem Molekulargewicht von 200 g/mol bis 20.000.000 g/mol, besonders bevorzugt mit einem Molekulargewicht von 5.000 g/mol bis 200.000 g/mol. Der Filmbildner wird vorzugsweise in Mengen von 0,01 Gew.% bis 30 Gew.-%, insbesondere 0,2 Gew.% bis 15 Gew.-% in den Mitteln, bezogen auf die Gesamtgewichtsmenge des Mittels, eingesetzt.

Eine besondere Form der Reinigung stellt die Desinfektion dar. Daher können die Mittel neben den Verbindungen der Formel (I) zusätzliche antimikrobielle Wirkstoffe enthalten.

Vorzugsweise werden als antimikrobielle Wirkstoffe Alkohole, Aldehyde, Carbonsäureester, Säureamide, Phenole, Phenolderivate, Diphenyle, Diphenylalkane, Harnstoffderivate, Sauerstoff-, Stickstoff-Acetale, sowie -Formale, Benzamidine, Isothiazole und deren Derivate, wie Isothiazoline und Isothiazolinone, Phthalimidderivate, Pyridinderivate, Guanidine, Chinoline, 1,2-Dibrom-2,4-dicyanobutan, Iodo-2-propynyl-butylcarbamat (IPBC), Iod, Iodophore und Peroxide eingesetzt. Besonders bevorzugt werden als antimikrobielle Wirkstoffe Ethanol, n-Propanol, i-Propanol, 1,3-Butandiol, Phenoxyethanol, 1,2-Propylenglykol, Glycerin, Undecylensäure, Citronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, quaternäre Verbindungen enthalten eine Ammonium-, Sulfonium-, Phosphonium-, Jodonium- oder Arsoniumgruppe, wie vorzugsweise Benzalkoniumchlorid, Peroxo-Verbindungen, insbesondere Wasserstoffperoxid, Alkalimetallhypochlorit, Dimethyldicarbonat und Guanidine eingesetzt.

Bevorzugt enthält das Mittel einen oder mehrere von Verbindungen der Formel (I) verschiedene antimikrobielle Wirkstoffe. Vorzugsweise enthält das Mittel einen oder mehrere antimikrobielle Wirkstoffe in einer Menge von 0,01 Gew.-% bis 1 Gew.-%, vorzugsweise in einer Menge von 0,02 Gew.-% bis 0,8 Gew.-% bezogen auf die Gesamtgewichtsmenge des eingesetzten Mittels.

In den erfindungsgemäßen Mitteln können gegebenenfalls wasserlösliche und/oder wasserunlösliche niedermolekulare Polycarbonsäuren, die homopolymeren und copolymeren Polycarbonsäuren und ihre Salze, die Citronensäure und ihre Salze, Carbonate, Phosphate und Silikate sein, die sogenannte Builder darstellen. Vorzugsweise enthalten die erfindungsgemäßen Mittel Citronensäure und ihre Salze, Carbonate, Phosphate und Silikate als Builder.

In einer weiteren Ausführungsform der Erfindung können die Mittel Bleichmittel enthalten. Vorzugsweise sind diese Bleichmittel Peroxide, Persäuren und/oder Perborate, besonders bevorzugt wird Wasserstoffperoxid als Bleichmittel eingesetzt.

In einer weiteren Ausführungsform der Erfindung können die Mittel Enzyme enthalten. Vorzugsweise werden als Enzyme Proteasen, Lipasen, Amylasen, Hydrolasen und/oder Cellulasen eingesetzt.

In einer weiteren Ausführungsform der Erfindung können die Mittel Konservierungsmittel enthalten. Als solche werden vorzugsweise die bei den antimikrobiellen Wirkstoffen genannten Stoffe eingesetzt.

Die erfindungsgemäßen Mittel werden vorzugsweise als vor der Anwendung entsprechend zu verdünnendes Konzentrat formuliert. Sie können durch Aufmischen unmittelbar aus ihren Rohstoffen, anschließendes Durchmischen und abschließendes Stehen des Mittels bis zur Blasenfreiheit hergestellt werden.

Von der Erfindung ist ebenfalls ein Verfahren umfasst, bei dem das erfindungsgemäße Mittel in einem Schritt

a.) mit einer Oberfläche in Kontakt gebracht wird und dann in einem Schritt b.) dass in Schritt a.) aufgetragene Mittel durch in Kontakt bringen der Oberfläche mit einem wässrigen Lösungsmittel wieder entfernt wird.

Das wässrige Lösungsmittel enthält bevorzugt > 90 Gew. % Wasser. Der Rest können organische, wie z.B. Alkohole oder andere anorganische Lösungsmittel sein. Das wässrige Lösungsmittel ist vorzugsweise Wasser.

In Schritt a.) wird das erfindungsgemäße Mittel vorzugsweise unter Zuhilfenahme eines Tuchs, eines Schwamms oder eines anderen geeigneten Substrats mit der Oberfläche in Kontakt gebracht und dort vorzugsweise verteilt.

In Schritt b.) kann das wässrige Lösungsmittel durch Eintauchen, Abwischen, Spülen oder Besprühen oder weiteren anderen geeigneten Verfahren mit der Oberfläche in Kontakt gebracht werden. Bevorzugt wird das wässrige Lösungsmittel durch Besprühen mit der Oberfläche in Kontakt gebracht.

Die erfindungsgemäßen Mittel werden vorzugsweise als Handseifen, Handgeschirrspülmittel, Maschinengeschirrspülmittel, Geschirrspülmaschinenreiniger, Waschmaschinenreiniger, Toilettenreiniger, WC-Reiniger, Universalreiniger, Allzweckreiniger, Küchenreiniger, Badreiniger, Sanitärreiniger, Fußbodenreiniger, Backofen- und Grillreiniger, Glas- und Fensterreiniger, Metallputzmittel, Polster und Teppichreiniger, Vollwaschmittel, Colorwaschmittel, Feinwaschmittel, Textilhilfsmittel, Vorbehandlungsmittel, Spezialwaschmittel und -reinigungsmittel, Mittel für die Textil- und Faserbehandlung und als Mittel für die Lederbehandlung eingesetzt.

Die erfindungsgemäßen Mittel werden vorzugsweise zur Reinigung harter Oberflächen verwendet. Harte Oberflächen im Sinne dieser Anmeldung sind dabei Fenster, Spiegel und weitere Glasoberflächen, Oberflächen aus Keramik, Kunststoff, Metall oder auch Holz sowie lackiertes Holz, die sich in Haushalt und Gewerbe finden, etwa Badkeramik,

Küchenoberflächen oder Fußböden. Insbesondere eignen sich die erfindungsgemäßen Mittel zur Fußbodenreinigung, zur Reinigung von Geschirr und zur Reinigung von Textilien.

Die erfindungsgemäßem Mittel zeigen hohe Reinigungsleistungen von Oberflächen auf. Sie sind besonders für die Verwendung als Reinigungsmittel für Geschirr geeignet.

Methoden

Bestimmung der kritischen Mizellkonzentration der Verbindungen der Formel (I) unter Verwendung der Pendant-Drop-Methode.

Die Pendant-Drop-Methode ist geeignet für Grenz- und Oberflächenspannungsmessungen. Dabei wird die Tropfengeometrie optisch erfasst; die Größe der Tropfen, die von einer Kapillare abtropfen, ist proportional zur Oberflächenspannung und erlaubt die Bestimmung der Oberflächenspannung. Technisch wird die Flüssigkeit aus einer Nadel injiziert, so dass sich ein Tropfen an der Spitze der Nadel bildet. Durch optische Kontrolle der Größe des Tropfen bzw. der Abtropfgeschwindigkeit bestimmt sich die Oberflächenspannung.

Dann wurden die gemessene Oberflächenspannung gegen die Konzentration der Verbindungen der Formel (I) aufgetragen und daraus dann die minimalste Oberflächenspannung und die zugehörige kritische Mizellkonzentration bestimmt. Die Konzentration, bei der die Oberflächenspannung $\sigma$ nicht weiter abnimmt, wird kritische Mizellenkonzentration genannt.

Vergleichende Bestimmung der kritischen Mizellenkonzentration der Verbindungen der Formel (I) unter Verwendung der Kibron Delta-8 Tensiometer.

Die vergleichende Messung der Oberflächenspannung wurde mit dem Kibron Delta-8 Tensiometer durchgeführt. Bei dieser Technik wird das Gewicht des Meniskus mit Hilfe eine empfindlichen Mikro-Waage ermittelt. Ein dünner Stift wird in die Probe eingelassen und beim hinausziehen die nötige Kraft gemessen (Du-Nouy-Padday Method, J. Chem. Soc., Faraday Trans. 1, 1975, 71, 1919 - 1931, DOI:10.1039/F19757101919), die nur von der Oberflächenspannung, dem Stiftdurchmesser und Dichte der Flüssigkeit abhängt.

Bestimmung der Hydrophilic-lipophilic balance (HLB) der Verbindungen der Formel (I)

Der HLB Wert beschreibt den Grad der Hydrophilie bzw. Lipophilie eines Stoffes. Es beschreibt die Änderung des chemischen Potentials eines oberflächenaktiven Stoffes beim Übergang von einer Öl- in eine Wasserphase.

Zur Messung des HLB Wertes wurde eine Mischung von Cyclohexane und Dimethicone als hydrophobe Phase verwendet. Der HLB Wert der Verbindungen der Formel (I) entspricht der Menge der hydrophoben Mischung von Cyclohexane und Dimethicone, welche zur stabilsten Emulsion führt, Diese errechnet sich wie folgt

$$A = (HLBneeded - HLBB) \div (HLBA - HLBB)$$

A = % Cyclohexane, HLBA= HLB Wert von Cyclohexane (=15) und HLBB= HLB Wert von Dimethicone (= 5).

## Beispiele

Folgende Mischung aus Verbindungen der Formel (I) wurden in den Beispielen 1 und 2 , sowie 4 eingesetzt:

| Verbindungen der Formel (I)<br>Nominal molecular weight [Da] | * |
|---|---|
| 886 (w/o acylation) | 0.4 % |
| 928 (1x acetyl) | 4.6 % |
| 954 | 5.8 % |
| 970 Verbindungen der Formel (V-B) | 41.9 % |
| 1012 Verbindungen der Formel (V-A) und (V-C) | 32.3 % |
| 1054 | 7.7 % |
| Verbindungen der Formel (V-D) und weitere Verbindungen der Formel (I) | 7.3 % |
| *relative Gewichtsprozente der eingesetzten Verbindungen der Formel (I) in der Mischung. | |

Die Gesamtmenge der eingesetzten Verbindungen der Formel (I) war 95 Gew.-% bezogen auf die Trockenmasse.

## Beispiel 1

Die Löslichkeit der Verbindungen der Formel (I) wurde in verschiedenen Lösungsmitteln, auch bei unterschiedlichen pH Werten, wie in Tabelle 1 aufgeführt, bestimmt.

Tabelle 1

| Lösungsmittel | Löslichkeit in % |
|---|---|
| Water, 25 °C or 50 °C | >5% |
| Water, 50 °C | >5% |
| Ethanol, 25 °C | 0.1% |
| Ethanol, 50 °C | 2.0% |
| 1,2-Propandiol, 85%, 25 °C | 2.0% |
| 1,2-Propandiol, 85%, 50 °C | 3.0% |
| 1,2-Propandiol, 50%, 25 °C | 0.1% |
| 1,2-Propandiol, 50%, 50 °C | 0.8% |
| Glycerin 85%, 25 °C | unlöslich |
| Glycerin 85%, 50 °C | 3.5% |
| Glycerin 50%, 25 °C | 1.5% |
| Glycerin 50%, 50 °C | 3.5% |
| Silicon oil, 25 °C or 50 °C | unlöslich |
| Mineral oil, 25 °C or 50 °C | unlöslich |
| Isopropyl palmitate, 25 °C or 50 °C | unlöslich |
| | |
| Cyclohexane, 25 °C | 70 ppm |
| Ethyl acetate, 25 °C | 0.01% |

(fortgesetzt)

| Lösungsmittel | Löslichkeit in % |
|---|---|
| Ethanol 90%, 25 °C | <1.0% |
| Ethanol 70%, 25 °C | <1.0% |
| Ethanol 50%, 25 °C | <1.0% |
| Ethanol 30%, 25 °C | <1.0% |
| Water, pH 2.5, 25 °C | 10 ppm |
| Water, pH 3.0, 25 °C | 30 ppm |
| Water, pH 3.5, 25 °C | > 50 ppm |
| Water, pH 4.0, 25 °C | > 50 ppm |

Die Verbindungen der Formel (I) sind gut in Wasser und Dimethylsulfoxid (DMSO) ab einem pH Wert von 5 löslich. Sie sind nur teilweise in Alkoholen und anderen organischen Lösungsmitteln wie Acetonitril löslich. Die Verbindungen der Formel (I) sind eher hydrophil als hydrophob.

**Beispiel 2**

Die Oberflächenspannung der Verbindungen der Formel (I) wurden nach der Pendant Drop Methode, bei unterschiedlichen Konzentrationen, bei Normaldruck und einer Raumtemperatur von 21 °C, bestimmt. Der pH-Wert der Lösung betrug ca. 6,0 und wurde nicht gepuffert.

Die Ergebnisse wurden in Tabelle 2 zusammengefasst.

Tabelle 2

| Konzentration [ppm] | σ [$10^{-3}$ N/m] | Zeitpunkt [s] |
|---|---|---|
| 0 | 72.75 | * |
| 1 | 52.26 | 590-610 |
| 2 | 53.20 | 590-610 |
| 3 | 50.94 | 590-610 |
| 7 | 45.98 | 290-310 |
| 15 | 42.67 | 190-210 |
| 31 | 40.56 | 140-160 |
| 62 | 39.89 | 140-160 |
| 125 | 40.43 | 80-90 |
| 250 | 40.48 | 140-160 |
| 1000 | 40.64 | 80-90 |

* Literaturwert bei 20°C (Chemielexikon, http://www.chemie.de/lexikon/Oberflächenspannung.html).

Die gemessene Oberflächenspannung wurden gegen die Konzentration der Verbindungen der Formel (I) aufgetragen und daraus dann die minimalste Oberflächenspannung und die zugehörige kritische Mizellkonzentration bestimmt.

Die kritische Mizellkonzentration (CMC) nach der Pendant Drop Methode beträgt 24 ppm.

Daraus folgt, dass $\Delta\sigma_{max}$ = 31,7 [$10^{-3}$ N/m], die berechnete minimalste Oberflächenspannung bei der kritischen Mizellkonzentration nach der Pendant Drop Methode ist.

Die kritische Mizellkonzentration von Tensiden bzw. Detergenzien im Vergleich zu den Verbindungen der Formel (I) sind in der Tabelle 3 aufgeführt.

Tabelle 3

| **Tenside (ausgewählte Glykolipide)** | $\Delta\sigma$ max [$10^{-3}$ N/m] | **CMC** (mg/l) |
|---|---|---|
| Sophorolipids (lactone form)* | 38.9 | 130 |
| Sophorolipids** | 35-60 / 26 | 5-80 |
| Rhamnolipids** | 27 / 33.8 | 110-150 |
| Mannosylerythritol lipids (MEL) AB** | 44-47 | 3.6x$10^{-4}$ Mol / 15.8x$10^{-4}$ Mol |
| Sodium laureth sulfate (SLES-3) | 42 | 74 |
| Dioctyl sodium sulfosuccinate (DOSS) | 46 | |
| Glykolipid der Formel (I) | 32 | 24 |
| * C.N.Mulligan, B.F.Gibbs, Proc. Indian natn Sci Acad. B70 No.1 pp 31-55 (2004);<br>**N. Lourith, M. Kanlayavattanakul, Int. Journal of Cosmetic Science, 2009, 31, 255-261 | | |

Verbindungen der Formel (I) weisen insbesondere hervorragende tensidische Eigenschaften auf und eigenen sich besonders als Detergenzien. Überraschend wurde jetzt gefunden, dass diese tensidischen Eigenschaften besser sind als bei den künstlichen und natürlichen Tensiden, insbesondere auch im Vergleich zu anderen Glykolipiden. Verbindungen der Formel (I) eignen sich daher besonders als Detergenzien bei geringen Konzentrationen. Insbesondere lassen sie sich hervorragend mit anderen Tensiden in einer Mischung einsetzen. Diese Mischungen zeigen besonders gute Reinigungseigenschaften, insbesondere von Gläsern, Keramiken und Porzellan auf. Zudem weisen die Verbindungen der Formel (I) zusätzlich konservierende Eigenschaften auf.

## Beispiel 3

Die Oberflächenspannung der Verbindung der Formel (V-B) im Vergleich zur Verbindung (IV) mit ((s=1, t=6, R1-7=OH) wurde getestet.

Die vergleichende Messung der Oberflächenspannung wurde mit dem Kibron Delta-8 Tensiometer durchgeführt bei unterschiedlichen Konzentrationen, bei Normaldruck und einer Raumtemperatur von 21 °C, bestimmt. Der pH-Wert der Lösung betrug ca. 6,0 und wurde nicht gepuffert.

Eine Serie von verschiedenen Konzentrationen der Glykolipid Verbindungen in deionisiertem Wasser mit 30% DMSO wurde ungepuffert bei Raumtemperatur gemessen.

Formel der Verbindung der Formel (IV) mit ((s=1, t=6, R1-7=OH)

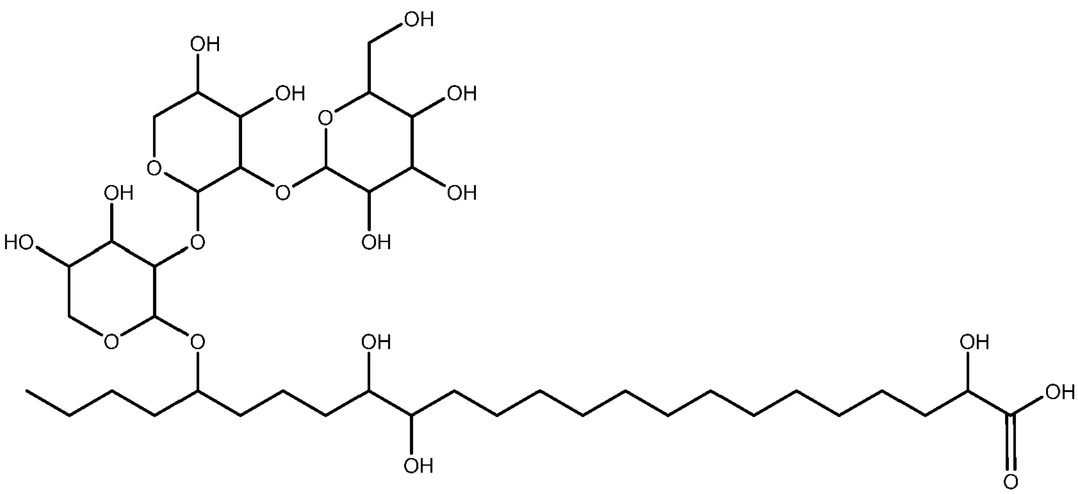

Die Ergebnisse sind in der Tabelle 4 dargestellt.

Tabelle 4

| Sample ID | CMC [mg/mL] | MaxPi [mN/m] |
|---|---|---|
| Verbindung der Formel (IV) mit (s=1, t=6, R1-7=OH) | 0.121 | 12.2 |
| Verbindung der Formel (V-B) | 0.066 | 16.9 |

Durch den Einsatz des organischen Lösungsmittels DMSO sinkt der CMC Wert ab gegenüber der Messung in Wasser. Überraschend weist die Verbindung der Formel (V-B) einen geringeren CMC Wert und größeren MaxPi Wert auf als die Verbindung der Formel (IV) mit (s=1, t=6, R1-7=OH).

## Beispiel 4

Bestimmung der Hydrophilic-Lipophilic Balance (HLB) der Verbindungen der Formel (I)
Insgesamt 15 Mischungen von Cyclohexan und Dimethicon wurden erstellt, und mit Verbindungen der Formel (I) versetzt, wie in Tabelle 5 gelistet. Die beste Emulsion wurde bei der Mischung 14 gefunden.

Tabelle 5

| HLBreq | | Cyclohexane /Dimethicone Ratio | | Water (g) | Oil Mix (g) | GLYCOLIPIDS (g) |
|---|---|---|---|---|---|---|
| | | | | | | |
| 5 | | 0/100 | | 5 | 0.005 | 0.005 |
| 6 | | 10/90 | | 5 | 0.005 | 0.005 |
| 7 | | 20/80 | | 5 | 0.005 | 0.005 |
| 8 | | 30/70 | | 5 | 0.005 | 0.005 |
| 9 | | 40/60 | | 5 | 0.005 | 0.005 |
| 10 | | 50/50 | | 5 | 0.005 | 0.005 |
| 11 | | 60/40 | | 5 | 0.005 | 0.005 |
| 12 | | 70/30 | | 5 | 0.005 | 0.005 |
| 13 | | 80/20 | | 5 | 0.005 | 0.005 |
| 14 | | 90/10 | | 5 | 0.005 | 0.005 |
| 15 | | 100/0 | | 5 | 0.005 | 0.005 |

Der gemessene HLB Wert von 14 bestätigt die sehr guten Eigenschaften der Verbindungen der Formel (I) als Reinigungsmittel.

Die Klassifizierung der Verbindungen der Formel (I) mit einem HLB Wert = 14 bedeutet, dass die Verbindungen der Formel (I) besonders als Detergenzien geeignet sind und aufgrund ihrer Hydrophilie insbesondere als Tensid in wässrigen Reinigungsmitteln (https://en.wikipedia.org/wiki/Hydrophilic-lipophilic_balance).

## Patentansprüche

1. Mittel enthaltend,

(i) mindestens eine Verbindung der Formel (I)

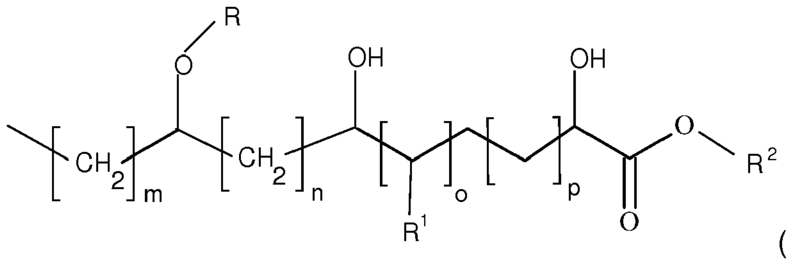

(I)

mit

m = 3 bis 5, bevorzugt 3 oder 5;
n = 2 bis 5, bevorzugt 3;
o = 0 oder 1 und
$R^1$ = H oder OH
$R^2$ = ist -H, Alkalimetall- oder Erdalkalimetallkation oder -$C_1$-$C_6$-Alkyl; vorzugsweise -H oder Alkalimetall- oder Erdalkalimetallkation und
p = 3 bis 17, bevorzugt 5 bis 15 wobei die Summe von m + n + o + p nicht kleiner als 14 ist und
R einen Kohlenhydratrest darstellt, der über ein Kohlenstoffatom an dem Sauerstoffatom gebunden ist

oder Gemische der Verbindungen der Formel (I) und
(ii) mindestens ein weiteres Tensid.

2. Mittel gemäß Anspruch 1, **dadurch gekennzeichnet, dass** der Kohlenhydratrest R eine Verbindung der Formel (III) ist

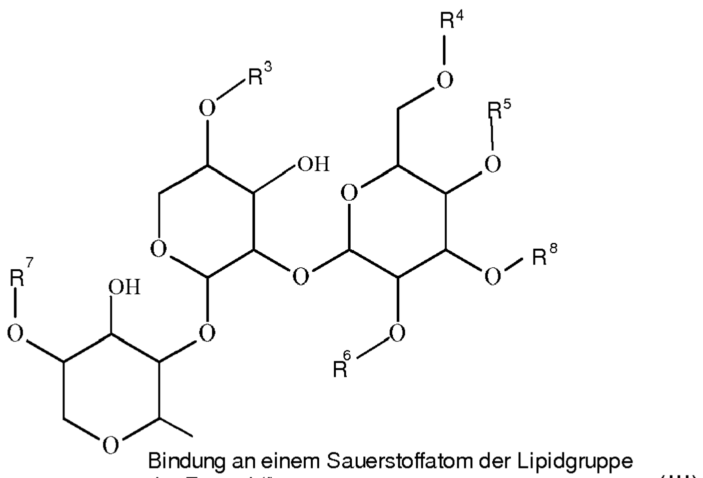

Bindung an einem Sauerstoffatom der Lipidgruppe der Formel (I)

(III)

wobei $R^3$, $R^4$, $R^5$, $R^6$ und $R^7$ unabhängig voneinander -H oder -C(=O)$C_1$-$C_{12}$-Alkyl sind und $R^8$= H, -C(=O)-$CH_2$-C(($CH_3$)(OH))-$CH_2$-COOH oder -C(=O)$C_1$-$C_{12}$-Alkyl ist; wobei $R^3$, $R^4$, $R^5$, $R^6$ $R^7$ und $R^8$ bevorzugt

unabhängig voneinander -H oder - $C(=O)C_1$-$C_6$-Alkyl sind ist, wobei $R^3$, $R^4$, $R^5$, $R^6$ und $R^7$ besonders bevorzugt unabhängig voneinander -H oder -$C(=O)C_3$-$C_6$-Alkyl und $R^8$ bevorzugt H oder - $C(=O)C_1$-$C_6$-Alkyl sind, noch weiter bevorzugt sind $R^4$ = -C(=O)isobutyl und $R^3$, $R^5$ , $R^6$, $R^7$ und $R^8$ unabhängig voneinander =-H oder/und -$C(=O)CH_3$.

3. Mittel gemäß Anspruch 1 oder 2, **dadurch gekennzeichnet, dass** die Verbindung Formel (I) einer Verbindung der Formel (IV) entspricht

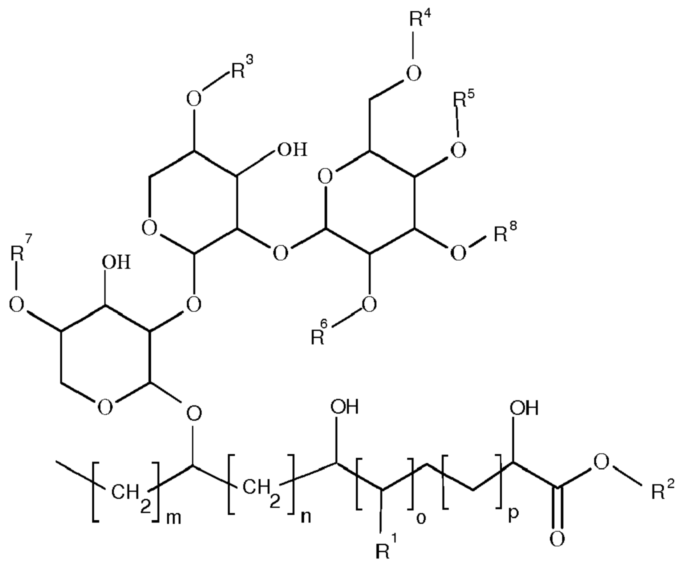

(IV)

wobei,

m = 4 oder 6,
n = 3,
o = 1
p = 11 oder 13
$R^1$ ist -H oder -OH, bevorzugt ist $R^1$ = OH,
$R^2$ ist -H, Alkalimetall- oder Erdalkalimetallkation oder -$C_1$-$C_6$-Alkyl; vorzugsweise - H oder Alkalimetall- oder Erdalkalimetallkation; und
$R^3$, $R^4$, $R^5$, $R^6$ und $R^7$ sind unabhängig voneinander -H oder -$C(=O)C_1$-$C_{12}$-Alkyl und $R^8$= H, -$C(=O)$-$CH_2$-$C((CH_3)(OH))$-$CH_2$-COOH oder -$C(=O)C_1$-$C_{12}$-Alkyl ist.

4. Mittel gemäß mindestens einem der Ansprüche 1 bis 3, **dadurch gekennzeichnet, dass** die Verbindung der Formel (I) mindestens eine Verbindung der Formel (V-A) bis (V-D) entspricht

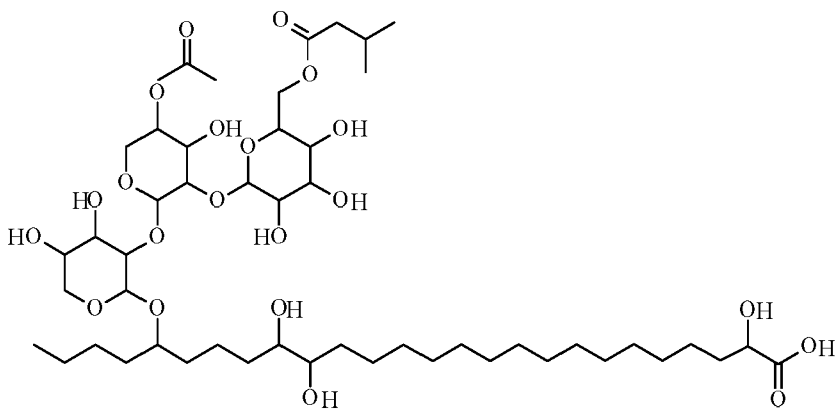

(V-A)

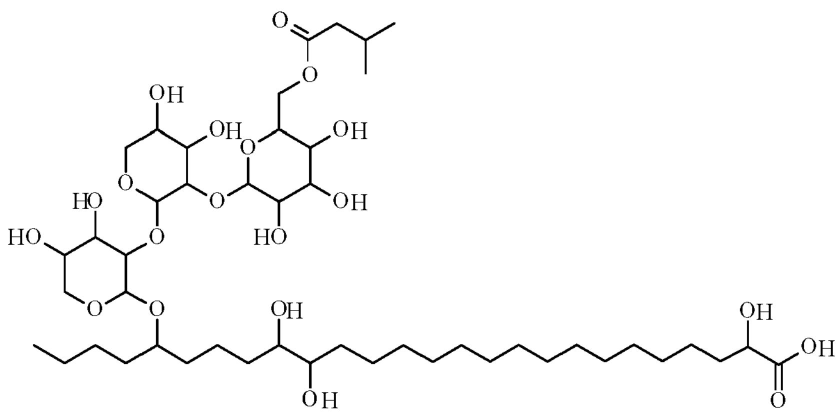

(V-B)

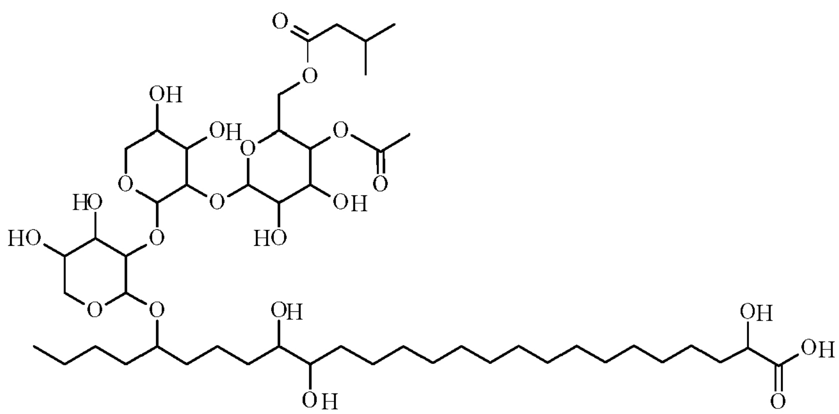

(V-C)

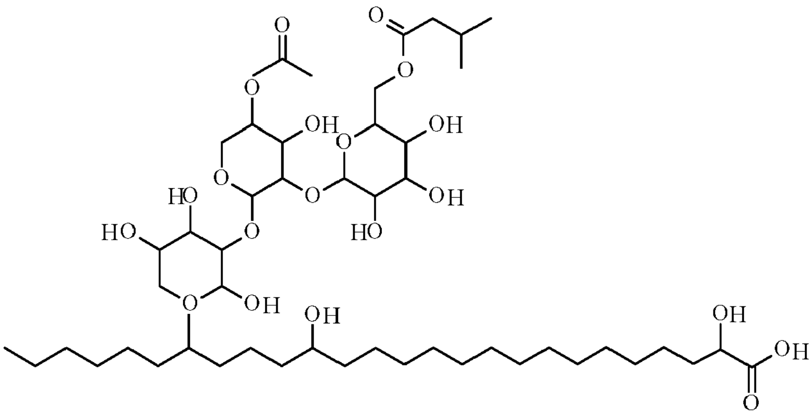

(V-D)

**5.** Mittel gemäß mindestens einem der Ansprüche 1 bis 4, **dadurch gekennzeichnet, dass** Gemische der Verbindungen der Formel (V-A) und Verbindungen der Formel (V-B) und Verbindungen der Forme (V-C) und Verbindungen der Formel (V-D) in einer Menge von 30 Gew. % bis 95 Gew. % bezogen auf die Gesamttrockenmasse der eingesetzten Verbindungen der Formel (I) eingesetzt werden.

**6.** Mittel gemäß mindestens einem der Ansprüche 1 bis 5, **dadurch gekennzeichnet, dass** als Tensid ii) mindestens ein Glykolipid ausgewählt aus der Gruppe Rhamnolipide, Sophorolipide, Mannosylerythriollipide, Cellobioselipide, Glucoselipide und Trehaloselipide eingesetzt wird.

**7.** Mittel gemäß mindestens einem der Ansprüche 1 bis 6, **dadurch gekennzeichnet, dass** als Tensid ii) mindestens ein anionisches Tensid ausgewählt aus der Gruppe $C_8$-$C_{18}$-Alkylbenzolsulfonate, $C_8$-$C_{20}$-Alkylsulfonate, $C_8$-$C_{18}$-Monoalkylsulfate (Fettalkoholsulfate), $C_8$-$C_{18}$-Alkylpolyglykolethersulfate mit 2 bis 6 Ethylenoxideinheiten (EO) im Etherteil, Sulfobernsteinsäuremono- und -di-$C_8$-$C_{18}$-Alkylester, $C_8$-$C_{18}$- -Olefinsulfonate, Dodecylbenzolsulfonat, $C_8$-$C_{22}$-Carbonsaureamidethersulfate, $C_8$-$C_{18}$-Alkylpolyglykolethercarboxylate, $C_8$-$C_{18}$-N-Acyltauride, N-Acylaspartate oder N-Acylglutamate, $C_8$-$C_{18}$-N-Sarkosinate und $C_8$-$C_{18}$-Alkylisethionate und deren Mischungen eingesetzt wird.

**8.** Mittel gemäß mindestens einem der Ansprüche 1 bis 7, **dadurch gekennzeichnet, dass** als Tensid ii) mindestens ein nicht-ionisches Tensid ausgewählt aus der Gruppe Fettalkoholpolyglykolether mit insbesondere 2 bis 8 Ethylenoxideinheiten, $C_{14}$-$C_{20}$-Aminoxide und $C_8$-$C_{10}$ Alkylpolyglucoside mit 1 bis 2 Glycosideinheiten und Mischungen dieser Verbindungen eingesetzt wird.

**9.** Mittel gemäß mindestens einem der Ansprüche 1 bis 8, **dadurch gekennzeichnet, dass** als Tensid ii) mindestens ein amphoteres Tensid der Formel $(R^i)(R^{ii})(R^{iii})N^+CH_2COO^-$ (VI) in der $R^i$ einen gegebenenfalls durch Heteroatome oder Heteroatomgruppen unterbrochenen $C_8$-$C_{25}$-Alkylrest ist, und $R^{ii}$ und $R^{iii}$ gleichartige oder verschiedene $C_1$-$C_3$-Alkyl sind, eingesetzt wird.

**10.** Mittel gemäß mindestens einem der Ansprüche 1 bis 9, **dadurch gekennzeichnet, dass** als Tensid ii) mindestens ein kationisches Tensid der Formel $(R^{iv})(R^{v})(R^{vi})(R^{vii})N^+X$- (VII) in der $R^{iv}$ bis $R^{vii}$ gleich- oder verschiedenartig sein kann, und unabhängig voneinander $C_8$-$C_{24}$-Alkyl und $C_1$-$C_6$-Alkyl sind und $X^-$ für ein einwertiges Anion, vorzugsweise ein Halogenid, steht.

**11.** Mittel gemäß mindestens einem der Ansprüche 1 bis 10, **dadurch gekennzeichnet, dass** das Tensid ii) in einer Menge von 1 bis 50 Gew. %, bevorzugt in einer Menge von 1,5 Gew. % bis 25 Gew. % bezogen auf die Gesamtgewichtsmenge des eingesetzten Mittels, in dem Mittel enthalten ist.

**12.** Mittel gemäß mindestens einem der Ansprüche 1 bis 11, **dadurch gekennzeichnet, dass** die Verbindungen der Formel (I) in einer Menge von 1 bis 50 Gew. %, bevorzugt in einer Menge von 5 Gew. % bis 40 Gew. % bezogen auf die Gesamtgewichtsmenge des eingesetzten Mittels, in dem Mittel enthalten sind.

**13.** Verwendung der Mittel gemäß mindestens einem der Ansprüche 1 bis 12 zur Reinigung von Oberflächen.

**14.** Verwendung der Mittel gemäß mindestens einem der Ansprüche 1 bis 12 als Handseifen, Handgeschirrspülmittel, Maschinengeschirrspülmittel, Geschirrspülmaschinenreiniger, Waschmaschinenreiniger, Toilettenreiniger, WC-Reiniger, Universalreiniger, Allzweckreiniger, Küchenreiniger, Badreiniger, Sanitärreiniger, Fußbodenreiniger, Backofen- und Grillreiniger, Glas- und Fensterreiniger, Metallputzmittel, Polster und Teppichreiniger, Vollwaschmittel, Colorwaschmittel, Feinwaschmittel, Textilhilfsmittel, Vorbehandlungsmittel, Spezialwaschmittel und - reinigungsmittel, Mittel für die Textil- und Faserbehandlung, Mittel für die Lederbehandlung und in kosmetischen oder dermatologischen Reinigungsmitteln für die Haut und im Zahnpflegebereich.

**15.** Verfahren zur Reinigung von Oberflächen, **dadurch gekennzeichnet, dass** das Mittel gemäß mindestens einem der Ansprüche 1 bis 12 in einem Schritt a.) mit einer Oberfläche in Kontakt gebracht wird und dann in einem Schritt b.) das in Schritt a.) aufgetragene Mittel durch in Kontakt bringen der Oberfläche mit einem wässrigen Lösungsmittel wieder entfernt wird.

Europäisches Patentamt
European Patent Office
Office européen des brevets

# EUROPÄISCHER RECHERCHENBERICHT

Nummer der Anmeldung
EP 18 21 0474

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | US 2014/178444 A1 (STADLER MARC [DE] ET AL) 26. Juni 2014 (2014-06-26)<br>* Extrakte 7 und 8;<br>Beispiele 14,16,17,18,19,21,22; Tabellen 24,24A; Verbindungen 1,4-10,12-14,16,18 *<br>----- | 1-15 | INV.<br>C11D1/66<br>A61K8/37<br>A61K8/99<br>C11D1/825<br>C11D1/83<br>C11D1/835 |
| | | | RECHERCHIERTE SACHGEBIETE (IPC) |
| | | | C11D<br>A61Q<br>A61K |

1 Der vorliegende Recherchenbericht wurde für alle Patentansprüche erstellt

| Recherchenort | Abschlußdatum der Recherche | Prüfer |
|---|---|---|
| München | 7. Mai 2019 | Culmann, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 18 21 0474

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-05-2019

| Im Recherchenbericht angeführtes Patentdokument | Datum der Veröffentlichung | Mitglied(er) der Patentfamilie | Datum der Veröffentlichung |
|---|---|---|---|
| US 2014178444 A1 | 26-06-2014 | BR 112013031371 A2 | 22-11-2016 |
| | | CA 2838442 A1 | 13-12-2012 |
| | | CN 103874411 A | 18-06-2014 |
| | | EP 2532232 A1 | 12-12-2012 |
| | | EP 2717691 A1 | 16-04-2014 |
| | | ES 2645257 T3 | 04-12-2017 |
| | | JP 6124878 B2 | 10-05-2017 |
| | | JP 2014516997 A | 17-07-2014 |
| | | KR 20140066989 A | 03-06-2014 |
| | | MX 350043 B | 24-08-2017 |
| | | MY 166455 A | 27-06-2018 |
| | | PL 2717691 T3 | 28-02-2018 |
| | | SG 195214 A1 | 30-12-2013 |
| | | SG 10201604666U A | 28-07-2016 |
| | | US 2014178444 A1 | 26-06-2014 |
| | | WO 2012167920 A1 | 13-12-2012 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2012167920 A
- EP 3290500 A
- WO 2015091250 A
- EP 2988829 B
- EP 2931237 B
- EP 0499434 B


### In der Beschreibung aufgeführte Nicht-Patentliteratur


- *CHEMICAL ABSTRACTS,* 15826-161
- *CHEMICAL ABSTRACTS,* 68515-73-1
- **DU-NOUY-PADDAY.** *Method, J. Chem. Soc., Faraday Trans. 1,* 1975, vol. 71, 1919-1931
- **C.N.MULLIGAN ; B.F.GIBBS.** *Proc. Indian natn Sci Acad.,* 2004, vol. B70 (1), 31-55
- **N. LOURITH ; M. KANLAYAVATTANAKUL.** *Int. Journal of Cosmetic Science,* 2009, vol. 31, 255-261